(19) Europäisches Patentamt

European Patent Office

Office européen des brevets

(11) **EP 0 909 953 B1**

(12) **EUROPEAN PATENT SPECIFICATION**

(45) Date of publication and mention
of the grant of the patent:
**27.12.2006 Bulletin 2006/52**

(51) Int Cl.:
*G01N 33/52* *(2006.01)*   *G01N 33/68* *(2006.01)*
*G01N 33/70* *(2006.01)*   *G01N 33/72* *(2006.01)*
*G01N 33/66* *(2006.01)*

(21) Application number: **98118706.5**

(22) Date of filing: **02.10.1998**

(54) **Method for improving the accuracy of the semi-quantitative determination of analyte in fluid samples**

Verfahren zur Verbesserung der Genauigkeit von der semi-quantitativen Bestimmung eines Analyts in flüssigen Proben

Méthode pour l'amélioration de la précision de la détermination semi-quantitative d'une analyte dans les échantillons fluides

(84) Designated Contracting States:
**BE CH DE ES FI FR GB IE IT LI NL SE**

(30) Priority: **14.10.1997 US 949520**

(43) Date of publication of application:
**21.04.1999 Bulletin 1999/16**

(73) Proprietor: **BAYER CORPORATION**
**Pittsburgh, PA 15205-9741 (US)**

(72) Inventors:
• **Messenger, Koleen K.**
**Granger,**
**Indiana 46530 (US)**
• **Pugia, Michael J.**
**Granger,**
**Indiana 46530 (US)**
• **Wallace, Jane F.**
**South Bend,**
**Indiana 46616 (US)**

(74) Representative: **Burkert, Frank et al**
**Bayer HealthCare AG**
**CAO Law and Patents**
**Patents and Licensing**
**51368 Leverkusen (DE)**

(56) References cited:
**EP-A- 0 263 401**        **EP-A- 0 617 285**
**EP-A- 0 895 084**        **WO-A-96/34271**
**US-A- 4 539 295**        **US-A- 5 108 896**
**US-A- 5 206 179**

• **HOEGHOLM, ASBJORN (1) ET AL:**
**"Microalbuminuria in 411 Untreated Individuals**
**With Established Hypertension, White Coat**
**Hypertension, and Normotension."**
**HYPERTENSION (DALLAS), (1994) VOL. 24, NO.**
**1, PP. 101-105. , XP000869529**

**Description**

**Background of the Invention**

Various analytical procedures and devices are commonly employed in assays to determine the presence and/or concentration of substances of clinical significance which may be present in biological fluids such as urine, whole blood, plasma, serum, sweat or saliva. Such substances are commonly referred to as analytes and can include specific binding partners, e.g. antibodies or antigens, drugs and hormones. One sort of test device is the so-called dipstick, containing enzymes which are interactive with the analyte and will interact with it in a manner which results in the oxidation of a redox dye to cause a color change which can be correlated with the presence or, in a semi-quantitative methods, the concentration of the analyte in the fluid sample. More recently there have been developed test strips which operate on the principle of immunochromatography in which labeled antibodies, specific for the analyte are applied to a strip of absorbant material through which the test fluid and labeled antibodies can flow by capillarity. By immobilizing analyte (or an analog thereof) in a particular portion of the strip, i.e. capture zone, and measuring the amount of labeled antibody which is captured through specific binding, the concentration of analyte in the test sample can be semi-quantitatively determined. This sort of assay, in which the label is an enzyme and there is placed a substrate for the enzyme in the capture zone to provide a colored response, is more fully described in U.S. Patent 4,446,232. In U.S. Patent 4,703,017 there is described a similar assay in which the label is a particulate material which, upon aggregation in the capture zone due to specific binding between the immobilized analyte and particle labeled antibody, provides a visible detectable response.

The clinical usefulness of analyses for various analytes can be enhanced by determining the concentration of a second analyte whose concentration in the biological fluid is clinically related to that of the first analyte. The most notable example of the second analyte is creatinine, the end metabolite when creatine becomes creatine phosphate which is used as an energy source for muscle contraction. The creatinine produced is filtered by the kidney glomeruli and then excreted into the urine without reabsorption. In order to increase the sensitivity of urinary assays and minimize the problem of high urine flow rates which result in urine dilution, analyte/creatine ratios are used in urine protein assays to normalize the urine concentration. Common creatinine assays include the alkaline Jaffe and Benedict-Behre methods which are run at a high pH, typically in the range of from 11.5 to 12.5. More recently, there has been developed a creatinine assay in which the urine sample is contacted with cupric ions in the presence of citrate, a hydroperoxide and an oxidizable dye which provides a colored response in the presence of oxygen free radicals and a pseudoperoxide. Creatinine quantitation may also be accomplished immunologically as described in WO 96/34271. Those second analytes whose concentration in the sample of body fluid is clinically related to the concentration of the first analyte are not limited to creatinine in urine nor is urine the only body fluid which can be assayed by the method of the present invention. Thus, for example, the body fluid tested can be whole blood and the first analyte can be $HbA_{1c}$ with the second analyte being total hemoglobin since the apparent concentration of $HbA_{1c}$ can be adjusted to the whole blood's total hemoglobin concentration to factor out bias in the $HbA_{1c}$ assay. Inulin, administered intravenously, is, like creatinine, an indicator of renal flow. Typical of other first analytes which can be assayed in conjunction with creatinine as the second analyte are occult blood, leukocytes, protein and glucose. The IgG concentration in urine can be corrected based on albumin as the second analyte.

In WO-96/34271 there is disclosed a device for determining a target (first) analyte and creatinine in a fluid test sample which device has an assay strip for the detection of creatinine and a second assay strip for the detection of the target analyte. The creatinine concentration may be determined colorimetrically or by the specific capture of labeled creatinine binding partners. The concentration of the target analyte is corrected based on the sample's concentration which correction can either be done manually or by means of a pre-programmed reflectance analyzer.

The prior art systems for correction of a determination of the concentration of a first analyte based on the concentration of the second analyte either involve directly ratioing the color response from the first analyte to that of the second analyte or first converting the color responses to concentration values and determining the ratio of these value arithmetically. The direct ratioing of the color responses is accomplished by converting color into numerical values such as absorbance or reflectance. These direct ratio methods for correction of semi-quantitative analyte determination results suffer from the limitation that they do not account for large errors in the estimates that occur when a method is reaching the end of its analytical range. The present invention increases the accuracy of semi-quantitative methods involving ratioing of two analytes by accounting for error in estimates that occur when a method is reaching the end of its analytical range.

**Summary of the Invention**

The present invention concerns a method as defined in claim 1.

## Description of the Invention

The first step in carrying out the present invention is to determine the uncorrected concentration of albumin. This can be accomplished by applying the fluid test sample to a test strip either directly to the portion of the strip containing the reagents as in the case of an enzymatic reaction or, in the case of an immunochromatographic strip, to a sample application pad in fluid communication with the capture zone of the strip, so that the sample can flow to the capture zone by capillary action. In either case the color change caused by analyte in the test fluid interacting with the strip's reagents can be read manually by comparing the color with a standard color chart or, more accurately, with the aid of a reflectance meter.

Once the uncorrected concentration of albumin determined, the next step is to ascertain if this concentration is within the useful analytical range for this analyte. The term useful analytical range indicates the concentrations of analyte that the method is capable of measuring with accuracy. The useful analytical range is 30 to 300 mg/L based on the error of the estimate being substantially smaller, i.e. at least three times smaller, than that the concentration of the analyte being estimated. If the determination of albumin as first analyte is within this range, then creatinine is determined and the ratio of albumin to creatinine i.e. [albumin]/[creatinine] is calculated to provide an output level whose value represents the corrected concentration of albumin in the urine test sample.

If the uncorrected concentration of albumin is outside the useful analytical range, e.g. less than 30 mg/L or greater than 300 mg/L albumin, then the normal concentration of creatinine is used to determine the ratio of albumin to creatinine. The term normal concentration is intended to mean the expected physiological value obtained with typical healthy patients. In the case of creatinine this value is 1,000 mg/L since the body typically excretes 1,000 mg of creatinine and 1 liter of urine per day. This is in contrast to prior art ratioing methods in which the determined, rather than normal, concentration of creatinine is used even in the cases where the concentration of the albumin is determined to be outside of the useful analytical range. The method of the present invention provides greater precision in determining the concentration of albumin because values which are inaccurately determined are not allowed to have the additional error added to the result.

The method of practicing the present invention is further illustrated by the following examples.

## General Example

In an analysis of urine in which albumin is determined and creatinine is used to correct for renal flow, a predetermined range of 30 mg/L to 300 mg/L albumin was assigned which is the useful analytical range for the urine albumin assay. If the albumin reagent produces a colorimetric result equivalent to 30 to 300 mg/L, an albumin to creatinine ratio is assigned using the ratio of colors produced (albumin color/creatinine color). If the albumin reagent produces a result of less than 30 mg/L or greater than 300 mg/L an albumin to creatinine ratio is assigned without use of the creatinine reagent by use of the normal creatine concentration. This prevents results from being used which are outside the analytical range of the albumin assay since results outside this range have large errors and are inaccurate. The results which are outside the analytical range are known with accuracy to be either less than 30 mg/L or greater than 300 mg/L which is medically important information.

If the albumin reagent produces a result between 30 mg/L and 300 mg/L, a ratio is assigned by dividing the result corresponding to the color formed by the albumin in the test sample with that corresponding to the color formed by the creatinine reagent. The ratio of color is then converted to the ratio of concentration of albumin to creatinine by assigning a specific degree of color ratio an output level which can be accomplished by a properly programmed reflectance spectrometer.

If the albumin concentration is less than 30 mg/L, there is assigned a threshold value of less than 30 mg/g which represents 30 mg albumin per gram of creatine. Less than 30 mg/g is considered a normal ratio for a healthy person. A lower result such as 20 or 10 mg/g does not change this since the threshold represents all values less than 30 mg/g. Conversely, if the albumin concentration is greater than 300 mg/L there is assigned a threshold value of greater than 300 mg/g which represents all values greater than 300 mg/g. The less than 30 mg/g and greater than 300 mg/g results are referred to herein as out of bounds results.

It is generally known that colors can only be measured within a certain absorbance range, typically less than 1.0 to greater than 0.1 absorbance units or greater than 10% to less than 99% reflectance. For this reason, spectrophotometers and reflectance meters are typically programmed to assign a color outside this range the nearest color which the meter is able to measure. For example, a 7% reflectance is read as 10% reflectance and it is this value that is used in the determination.

A sample calculation is presented in Table 1 which demonstrates that the present method has a greater agreement of the albumin and creatinine reagent than the ratio obtained from two prior art methods.

### TABLE 1

| Ratio Method | Total Number of Correct Results | Remarks |
|---|---|---|
| Division | 70% | as in Ex. 1 |
| Color ratio method | 79% | as in Ex. 2 |
| Color ratio method with cut-off for out of bounds results | 95% | as in Ex. 3 |

From Table 1, it can be determined that greater assay accuracy can be obtained with the method of the present invention than with simple division or with a color ratio method that does not delete out of bounds results.

The following examples involve urine strip reagents which typically involve colorimetric assays whose color is read visually or instrumentally as reflectance or absorbance. The color produced is directly proportional to the analyte concentration. In the case of albumin, the more albumin reagent color formed, the more albumin is present in the urine specimen. In order to convert color to analyte concentration, a specific degree of color is assigned an output level. The output levels of an analyte are assigned a concentration range representing the typical error of the estimation. This is common practice for all urine reagent strips and is shown for albumin and creatinine reagents in the following Table 2. For example, a clinical specimen with a value of 30 mg/L albumin by a standard method could be 20 to 39 mg/L as determined by an albumin strip color but would still be assigned an albumin concentration of 30 mg/L. The smaller the error of the estimation, the more quantitative the method.

### TABLE 2

| output Value | Albumin (mg/L) Expected Concentration Range | output Value | Creatinine (mg/dL) Expected Concentration Range |
|---|---|---|---|
| 0 | 0-20 | 30 | 0-64.9 |
| 30 | 20-39.9 | 100 | 65.0-149.9 |
| 80 | 40-119.9 | 200 | 150.0-249.9 |
| 150 | 120-199.9 | 300 | 250.0-350.0 |

**Example 1** - Prior Art Method to Ratio Two Analytes

The most common method to ratio two analytes is to use a look-up table (Table 3 below).

### TABLE 3

| mg Albumin gram Creatinine Ratio Table: | | Assigned output | | |
|---|---|---|---|---|
| Creatinine | 0 mg/L | 30 mg/L | Albumin 80mg/L | 150 mg/L |
| 30 mg/dL | <30 mg/g | 30-299 mg/g | 30-299 mg/g | 300 mg/g |
| 100 mg/dL | <30 mg/g | 30-299 mg/g | 30-299 mg/g | 30-299 mg/g |
| 200 mg/dL | <30 mg/g) | <30 mg/g) | 30-299 mg/g | 30-299 mg/g |
| 300mg/dL | < 30 mg/g | <30 mg/g | <30 mg/g | 30-299 mg/g |

Each combination of strip outputs is assigned an expected ratio output. The expected output ratio is based on the division of the mean result of each strip as shown in Table 4.

### TABLE 4

| mg Albumin/ gram Creatinine Ratio Table: | | Mean value of dividing each strip | | |
|---|---|---|---|---|
| Creatinine | 0 mg/L | 30 mg/L | Albumin 80 mg/L | 150 mg/L |
| 30 mg/dL | 0 | 100 | 267 | 500 |
| 100 mg/dL | 0 | 30 | 80 | 150 |
| 200 mg/dL | 0 | 15 | 40 | 75 |
| 300 mg/dL | 0 | 10 | 27 | 50 |

This method introduces error, since each mean output result represents an expected range and the extremes of the expected ranges do not always agree with the assigned output. For example, 30 mg/L albumin has a low extreme of 19.9 mg/L and 100 mg/dL creatinine has a high extreme of 150 mg/dL. The expected ratio output of these extremes is 13 mg/g which is not in the 30-299 mg/g assigned range. This erroneous assignment would be an incorrect ratio result even if the reagents agreed 100% with the standard methods.

The error in this method is shown in the following truth table in which the strip results are compared to standard values of 275 clinical specimens. The range of clinical specimen assigned a given albumin to creatinine strip output is much greater than the expected concentration range which makes this method inaccurate and ineffective. The total number of correct results for two levels of output, i.e. less than 30 mg/g and greater than 30 mg/g, is 70% (86 and 109 out of 225) with more than 35% of the >30 mg/g specimens being incorrectly assigned as can be determined by the 76 results out of 185 total results which are greater than 30 mg/g by the strip are less than 30 mg/g by the standard method.

Truth Table for Ratioing by Method of Example 1

| Standard methods | Albumin | | |
|---|---|---|---|
| | creatinine dipstick ratio | | |
| | <30 mg/g | >30 mg/g | total |
| <30 mg/g | 86 | 76 | 162 |
| >30 mg/g | 4 | 109 | 113 |
| total | 90 | 185 | 275 |

**Example 2** - Another common method to ratio two analytes known in the prior art is to convert the result of each individual reagent to concentrations of the analyte to be detected. This conversion is done by comparing a standard specimen having a known concentration to the unknown specimen and assigning to the unknown a concentration relative to the color differences of the two specimens. The analyte concentrations can then be divided to produce a ratio of concentration. This approach is very common with colorimetric assay methods that have a high degree of accuracy. However, using this method with colorimetric assay methods having a lower degree of accuracy, for example urine dipstick methods, has a disadvantage since they have a smaller analytical range than solution methods which can make dilutions and timed addition of reagents to limit error increasing interferences. These quantitative methods have analytical ranges which extend beyond the concentrations which are expected to be encountered.

The results of this method of ratioing, as shown in the following Table 5, are only slightly better than the method discussed in Example 1. The total number of correct results for two levels of output is

$$79\% \ (\underline{125 \ + \ 93} \ = \ 0.79).$$
$$275$$

TABLE 5

Truth table for Ratioing by Method of Example 2

| Standard methods | Albumin | | |
|---|---|---|---|
| | creatinine dipstick ratio | | |
| | <30 mg/g | ≥30 mg/g | total |
| <30 mg/g | 125 | 37 | 162 |
| ≥30 mg/g | 20 | 93 | 113 |
| total | 145 | 130 | 275 |

## Example 3

The ratioing method of the present invention uses the divided result of the color formed by two reagents in combination with the colors formed by the individual reagent.

Initially, the color of the reagent which is sensitive to albumin is converted to the concentration of albumin i.e. a specific degree of color is assigned an output value. The output level is examined and if the level is outside the useful analytical range, it is used to assign a ratio by using the normal value of creatinine. For example, an albumin reagent producing a result of less than 30 mg/L or greater than 300 mg/L of urine is assigned an output ratio without reference to the creatinine reagent result and instead using the normal creatine value at 1,000 mg/L. Albumin at a concentration of less than 30 mg/L is assigned a ratio of <30 mg/gm and albumin at greater than 300 mg/L is assigned a ratio of >300 mg/gm. This assignment assumes average creatinine excretion of 1,000 mg/L. If the output of the first analyte is inside the useful analytical range, the color formed by the reagent which is sensitive to the first analyte is divided by the color formed by the reagent which is sensitive to the second analyte. The ratio of colors is then converted to a ratio concentration as in Table 6.

TABLE 6

| Output Value* | Color Ratio |
|---|---|
| <30 | <1.5 |
| 30-300 | 1.5 to 3 |
| >300 | >3 |

*mg albumin/g creatinine

For example, an albumin reagent producing a result of 80 mg/L is assigned an output ratio based on the color ratio of the albumin and creatine reagents, in which case a color ratio of 1.7 would be assigned an output ratio of 30-300 mg/g.

The following Table 7 shows that this method has a greater agreement than either of the previously described methods. The greater agreement is due to excluding the greater error often observed with a reagent at the ends of its output range, i.e. past the lowest or highest output levels. Results outside the analytical range have large errors and are inaccurate. Either or both the lowest and highest output levels can be excluded for any reagent being ratioed. In the Table 7, the total number of correct results for two levels of output, i.e. less than 30 mg/g or greater than 30 mg/g is 95%. This can be determined by the number of specimens correctly determined (149 + 112) divided by 275 which is the total number of specimens.

TABLE 7

Truth table for Ratioing by Method of Example 3

| Standard methods | Albumin creatinine dipstick ratio | | |
|---|---|---|---|
| | <30 mg/g | ≥30 mg/g | total |
| <30 mg/g | 149 | 13 | 162 |
| ≥30 mg/g | 1 | 112 | 113 |
| total | 150 | 125 | 275 |

**Claims**

1. An analytical method for the analysis of a sample of urine for the concentration of albumin using the concentration of creatinine to correct the concentration of albumin which comprises:

   a) determining the observed concentrations of albumin and creatinine using a quantitative analytical procedure for each;
   b) determining if the observed concentration of albumin is inside or outside the threshold limit 30 mg/L to 300 mg/L which are within the dynamic ranges of the quantitative analytical procedure;
   c) if the uncorrected concentration of albumin is less than 30 mg/L or greater than 300 10 mg/L then dividing the uncorrected concentration by 1,000 mg/L to obtain a ratio representing the corrected albumin concentration and
   d) if the value of the albumin is between the threshold limit, then assigning a ratio value by dividing the color formed by the albumin in the test sample with the corresponding color formed by the creatinine reagent.

**Patentansprüche**

1. Analyseverfahren zur Analyse einer Urinprobe auf die Konzentration von Albumin unter Verwendung der Konzentration von Kreatinin zur Korrektur der Albuminkonzentration, Folgendes umfassend:

a) das Bestimmen der beobachteten Konzentrationen von Albumin und Kreatinin, jeweils unter Anwendung eines quantitativen Analyseverfahrens;

b) das Bestimmen, ob die beobachtete Konzentration von Albumin innerhalb oder außerhalb der Grenzwerte 30 mg/l bis 300 mg/l liegt, die im Dynamikbereich des quantitativen Analyseverfahrens liegen;

c) wenn die unkorrigierte Albuminkonzentration unter 30 mg/l oder über 300 mg/l liegt, das Dividieren der unkorrigierten Konzentration durch 1.000 mg/l, um ein Verhältnis zu erhalten, das die korrigierte Albuminkonzentration darstellt, und

d) wenn der Albuminwert innerhalb der Grenzwerte liegt, das Zuordnen eines Verhältniswerts durch Dividieren des durch das Albumin in der Probe bedingten Farbwerts durch den entsprechenden durch das Kreatininreagens bedingten Farbwert.

## Revendications

1. Méthode analytique pour l'analyse d'un échantillon d'urine pour la détermination de la concentration d'albumine en utilisant la concentration de créatinine afin de corriger la concentration d'albumine, qui comprend les étapes consistant :

a) à déterminer les concentrations observées d'albumine et de créatinine en utilisant un mode opératoire analytique quantitatif pour chacune ;

b) à déterminer si la concentration observée d'albumine est située à l'intérieur ou à l'extérieur des valeurs limites seuil de 30 mg/l à 300 mg/l qui sont comprises dans les intervalles dynamiques du mode opératoire analytique quantitatif ;

c) si la concentration non corrigée d'albumine est inférieure à 30 mg/l ou supérieure à 300 mg/l, alors à diviser la concentration non corrigée par 1000 mg/l pour obtenir un rapport représentant la concentration d'albumine corrigée, et

d) si la valeur de concentration d'albumine est comprise dans les limites seuils, alors à attribuer une valeur de rapport en divisant la couleur formée par l'albumine dans l'échantillon d'essai par la couleur correspondante formée par le réactif pour la créatinine.